# EUROPEAN PATENT APPLICATION

(11) **EP 4 368 131 A1**
(43) Date of publication of application: **15.05.2024**
(21) Application number: 23198532.6
(22) Date of filing: 20.09.2023
(51) Int. Cl.: A61B 18/14, A61B 18/00, A61B 17/00

(54) **HAND-HELD ELECTROSURGICAL INSTRUMENT, INSULATING INSERT AND ELECTRODE SUPPORT FOR HAND-HELD ELECTROSURGICAL INSTRUMENT**

(30) Priority: 09.11.2022 US 202263423992 P
(71) Applicant: Olympus Winter & Ibe GmbH, 22045 Hamburg (DE)
(72) Inventor: Knopf, Christoph, 23617 Stockelsdorf (DE); Brockmann, Christian, 21279 Hollenstedt (DE); Lavicka, Jiri, 75002 Prerov (CZ); Komínek, Petr, 75002 Prerov (CZ)
(74) Representative: Hoener, Matthias

(57) **Abstract**

The invention provides an insulating insert, an electrode carrier, and a hand-held electrosurgical instrument that can be used in a particularly efficient manner and can be manufactured in a particularly cost-effective manner. This is achieved in that the insulating insert (29) is of tubular design and can be releasably coupled by a proximal end region to a distal end of a tubular shaft (13) of the hand-held instrument, and a central passage (36) serves to receive an inner shaft of the hand-held instrument, wherein two bores (37, 38) for receiving a respective electrode carrier tube (25, 26) of an electrode carrier (14) are arranged in a wall (35) of the insulating insert (29) opposite and parallel to the central passage (36).

## Description

The invention relates to an insulating insert for an electrosurgical hand-held instrument according claim 1, and to an electrode support for an electrosurgical hand-held instrument according to claim 5. In addition, the invention relates to a method of manufacturing an electrode support according to claim 14, and to an electrosurgical hand-held instrument according to claim 16.

Electrosurgical hand-held devices, in particular resectoscopes, of the type described are used primarily in urology for electrosurgical work. In this context, these devices are usually used for resection and for evaporation of tissue, for example of tissue in the lower urinary tract. For this purpose, the hand-held device, in particular the resectoscope, may comprise a longitudinally displaceable electrode support which, after insertion of the device into the body to be treated, may be advanced with a distal working end from a distal end of the instrument shaft of the hand-held device. An electrosurgical electrode is disposed on the electrode support at a distal end thereof. This electrode may be in the form of a loop, for example, and is pulled or pushed through the tissue to manipulate the tissue, depending on the design of the instrument.

For the above application, a high-frequency electric current is applied to the electrode. It is important to avoid the electrode coming into electrical contact with the shaft tube of the handpiece. In the event of such electrical contact, a short circuit could cause a device defect or lead to unpredictable trauma in the body to be treated. To prevent such short circuits, handheld devices include an electrically insulating insulating insert; also called an insulating tip, at their distal end region. The insulating insert can be attached either to an inner shaft or shaft tube, in which an electrode support is guided, or to the outer shaft of the instrument. Since such hand-held instruments can also be designed for multiple use and must therefore be regularly sterilized or autoclaved, the insulating insert is designed to be detachable for cleaning.

In the case of the hand instrument described here for minimally invasive treatment of patients, the aim is for the size of the instruments or their cross-section to be as small as possible in order to minimize trauma to the patient during treatment. Similarly, the aim is to perform the procedure in a particularly efficient manner. The choice of electrode is of crucial importance for efficient surgery. Only with the right, application-specific electrode can the optimal treatment goal be achieved. In particular, the effective cross-section of the electrode or the working instrument relative to the cross-section of the instrument can be of decisive importance. However, the effective cross-section or size of the electrode is limited by the shape and diameter of the shaft of the electrosurgical hand instrument. Thus, it is impractical for the effective cross-section of the electrode to be larger than the cross-section of the outer circumference of the shaft. However, in known instruments, the space available for the electrode is not optimally utilized. Approaches for better utilization of the available space pursue highly complicated electrode geometries, which on the one hand are very complex and thus expensive to manufacture, and on the other hand require a great deal of effort in quality control.

The invention is therefore based on the problem of creating an insulating insert, an electrode support and a hand-held electrosurgical instrument that can be used particularly efficiently and can be manufactured in a particularly cost-effective manner.

A solution to this problem is described by claim 1. Accordingly, it is provided that the insulating insert according to the invention is tubular and is detachably couplable with a proximal end portion to a distal end of a tubular shaft of the hand-held device and a central passage serves to receive an inner shaft of the hand-held device. This inner shaft may be a shaft for receiving a tool or optics. According to the invention, it is provided that two bores are arranged in a wall of the insulating insert, opposite and parallel to the central passage, for receiving in each case an electrode support tube of an electrode support. By receiving the electrode support tubes through the two holes in the insulating insert, the stability of the electrode support and thus also of the electrode is improved. By supporting the distal end regions of the electrode support in the bores, the stability of the at least one electrode support transverse to a longitudinal axis of the hand instrument is improved. This guidance of the electrode support in the bore allows the electrode to be inserted in a particularly precise and thus efficient manner.

In particular, it is provided that the two bores have an oval cross-section, wherein a height of the oval cross-section is greater than a width of the oval cross-section. Furthermore, it is preferably provided that the two bores are displaced upwards relative to a central longitudinal axis or central bore. The oval configuration of the bores, as well as the upward or downward displacement from the central position of the bores, also displaces the positions of the electrode support tubes passing through the insulating insert and through the hand instrument. This shifting and the oval design of the bores allow electrodes to be inserted that have a greater height than previous electrodes while maintaining the same width. This design of the holes allows electrodes to be used with a larger effective cross-section relative to the cross-section of the insulating insert.

A further advantageous embodiment of the invention may provide that the wall of the insulating insert is reinforced around the two bores and is otherwise reduced in wall thickness. The insulating insert, which is otherwise thin-walled, has two inwardly pointing bulges in the region of the bores, by means of which the inner, open cross-section of the insulating insert is minimally reduced. Space can be created in the central interior of the insulating insert in particular by shifting these bulges upwards out of the central edge area.

According to the invention, it can be provided that the insulating insert, which is formed of an electrically insulating material, such as a temperature- and plasma-stable plastic, has coupling means to be detachably connected to the shaft. During assembly of the hand-held instrument, the electrode support with the two electrode support tubes is passed through the holes and then releasably attached to the distal end of the shaft. The proximal ends of the electrode support tubes can be connected to a main body of the hand instrument. For disassembly of the hand instrument, the above steps are performed in the reverse order.

An electrode support for solving the aforementioned problem has the features of claim 5. Accordingly, the electrode support for an electrode of an electrosurgical hand instrument has at least one, preferably two, electrode support tubes, wherein the electrode can be arranged at a distal end of the at least one electrode support tube. This electrode support can be coupled to a main body of the hand instrument via a proximal end of the at least one electrode support tube. Via this main body, the electrode support can be moved axially as well as subjected to a high-frequency electric current. According to the invention, it is provided that a cross-section of the at least one electrode support tube is oval or convex, in particular over an entire length. This oval design of at least one section of the electrode support tube leads to increased stability with respect to forces acting on the electrode support tube transversely to a longitudinal axis of the hand instrument. Furthermore, the oval shape leads to a reduction in the space required by the at least one electrode support tube within an instrument shaft. Furthermore, the position of the distal ends of the at least one electrode support tube and thus of the electrode can be optimized.

Preferably, a distal portion of the at least one electrode support tube has an oval or convex cross-section. The remaining portion of the electrode support tube may further have a circular or arbitrary cross-section. In this regard, it is provided that the at least one electrode support tube is oriented such that a height of the oval cross-section is greater than a width of the cross-section, wherein the height is a dimension perpendicular to a horizontal plane.

Preferably, the height-to-width ratio is 1.1:1 to 1.7:1, preferably 1.4:1, over the entire length or only over the distal section. It has been shown that this dimensioning of the at least one electrode support tube can be positioned within the instrument shaft in a particularly space-saving manner. This width-to-height ratio represents an optimal compromise between stability and repositioning of the electrode at the distal end of the electrontractor. As a preferred dimension of the height, 1.4 mm may be mentioned and for the width 1 mm. However, it is also provided according to the invention that the absolute dimensions of the oval cross-section deviate at least slightly from these values. Due to the oval shape of the cross-section, the at least one electrode support tube can be moved upwards from the center of the instrument without changing the distance between the two distal ends of the electrode support tubes. The oval shape allows the distal end portions of the tubes to be moved upward relative to the central axis of the hand instrument while maintaining the same spacing, such that the loop size of the electrode is increased, all without protruding beyond the cross-section of the hand instrument. This reshaping of the electrode support tubes thus makes it possible to work with an electrode that has an optimized working cross section.

A preferred embodiment of the invention provides that the distal section of the at least one electrode support tube has a length of from 20 mm to 50 mm, preferably from 24 mm to 40 mm, in particular is longer than 30 mm. Thereby, just this distal section has an oval cross-section. The length of the section corresponds at least to the stroke length of the electrode support within the instrument shaft.

Preferably, the invention may further provide that a transition between a proximal portion of the at least one electrode support tube and the distal portion is formed by a crimp. This crimping represents a local reshaping of the outer circumference of the electrode support tube into a hexagonal cross-section. Accordingly, the diameter of the electrode support tube is reduced in certain areas. This forming of the tube serves a defined transition from the tube section with a circular to the tube section with an oval cross section. In addition, the forming serves to fix the electrical conductor or wire within the tube.

The hexagonal crimp of the at least one electrode support tube is oriented such that two opposite side surfaces of the hexagonal cross-section are parallel to each other as well as perpendicular to a horizontal plane. Due to this orientation of the crimp, the maximum diameter of the crimped tube section does not exceed the oval diameter of the distal section, so that snagging of the electrode support cannot occur during back and forth movement relative to the longitudinal axis of the instrument.

In addition, it is preferably provided that a distal end of the at least one electrode support tube also has a preferably hexagonal crimp. By means of this crimping, the interior of the tube can be sealed in a watertight manner and thus the internal wire can be protected against inflowing liquids. It may be provided that this hexagonal forming of the cross-section is also aligned in the same way as the crimping between the section with the round cross-section and the section with the oval cross-section.

Another particularly preferred embodiment of the invention may provide that the at least one electrode support tube has at least one, preferably two, namely a proximal S- curvature and a distal S- curvature, S- curvature, wherein by said at least one S- curvature a portion of the electrode support tube is displaced in parallel relative to another portion of the electrode support tube. This S- curvature allows the distal end, to which the electrode is attached, of the at least one electrode support tube to be displaced relative to the central axis through the instrument. This displacement of the distal end portion is particularly possible because the distal end portion has the oval cross-section described above. Thus, it is possible that the S- curvature needs to be performed in only one dimension, rather than in two. Accordingly, the relative distance between the two distal ends of the parallel guided electrode support tubes does not change as a result of the S- curvature. Only the two distal ends are shifted upward relative to the central axis. Due to the changed cross-section of the distal end region, this does not lead to a collision of the electrode support tubes with the instrument shaft. This displacement of the distal ends allows electrodes to be used which have a larger effective cross-section without protruding beyond the cross-section of the instrument shaft.

Preferably, it is provided that the at least one S-curvature, in particular the distal S- curvature has a height of 0.2 mm to 2 mm, preferably 0.7 mm. The length of the at least one S-curvature may be from 2 mm to 20 mm, preferably from 5 mm to 10 mm.

Furthermore, it may be provided according to the invention that the distal section of the at least one electrode support tube with the oval or convex cross-section has at least one distal S-curvature. In the case that the at least one electrode support tube has two S-curvatures, namely a distal and a proximal S-curvature, it may be provided that these two S-curvature lie in a common plane. Similarly, it is also conceivable that the two planes of the S-curvatures are twisted with respect to each other.

A method for solving said problem has the measures of claim 14. Accordingly, the method of manufacturing an electrode support for an electrode of an electrosurgical hand instrument having at least one, preferably two, electrode support tubes as claimed in claim 5, comprises forming a cross-section of a distal portion of the electrode support tube oval (step A) and/or crimping a transition from a portion having a circular cross-section to a portion having an oval cross-section (step B) and/or providing the distal region having the oval cross-section with at least one S- curvature (step C). According to the invention, it is provided that steps A, B and C are carried out successively or simultaneously.

A hand-held electrosurgical device for solving the aforementioned problem has the features of claim 16. In particular, the hand-held electrosurgical device may be a resectoscope or the like. The hand-held device has a main body to which a tube-like shaft is connected. An insulating insert according to claims 1 to 4 is disposable at a distal end of this shaft, wherein an electrode support according to claims 5 to 13 extends through the shaft and through the insulating insert and is attached to the main body with a proximal end. An electrode is arrangeable at the distal end of the electrode support or electrode support tubes.

A preferred embodiment of the invention is described in more detail below with reference to the drawing. In this show:
- Fig. 1: a schematic representation of a handheld surgical device, in particular a resectoscope,
- Fig. 2: a perspective view of an electrode support,
- Fig. 3: a side view of a distal section of the electrode support according to Fig. 2,
- Fig. 4: a view of an insulating insert, and
- Fig. 5: a view of the insulating insert according to Fig. 4 with the electrode support,

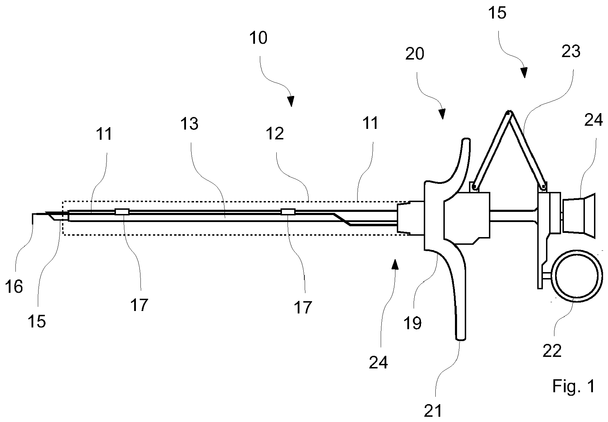

Fig. 1 shows a schematic, side cross-sectional view of a resectoscope known as 10. The resectoscope 10 has a resectoscope shaft 11, which includes an outer shaft 12 or sheath tube shown. A tubular inner shaft 13 extends within the outer shaft 12. A electrode array 14 and an indicated optic 15 are shown within the inner shaft 13. In addition, other elements not shown here may be disposed within the resectoscope 10, such as a separate irrigation tube and the like.

The electrode array 14 has an electrosurgical tool or electrode 16 at a distal end. The electrode 16 shown here is shown as a loop, but may also be formed as a button or the like.

The electrode holder 14 can be forcibly moved axially in the distal and proximal direction by actuating a handle 19. In doing so, it can be pushed beyond the distal end of the inner shaft 13 and the outer shaft 12. This allows the surgeon to manipulate tissue further away from the resectoscope tip. Furthermore, for this purpose, the inner shaft 13 and/or the electrode support 14 can be rotatably mounted about their longitudinal axis. For the manipulation of the tissue, a high-frequency electric current is applied to the electrode 16.

The resectoscope 10 shown in Fig. 1 has a passive transporter in which a carriage 20 is displaced in the distal direction against the distal, first handle part 21 by relative movement of the handle parts 21 and 22 arranged proximally on the resectoscope shaft 11 against a spring force applied by a spring bridge 23. When the carriage 20 is displaced in the distal direction against the handle part 21, the electrode support 14 is displaced in the distal direction in a manner not shown. When the load on the handle parts 21, 22 is relieved, the spring force generated by the spring bridge 23 forces the slide 20 back to its initial position, pulling the electrode support 14 in the proximal direction. When the slide 20 is moved back, an electrosurgical procedure can be performed with the electrode 16 without any manual force from the operator, i.e. passively.

For targeted treatment by means of the electrode 16, the optics 15 are positioned in such a way that the surgeon has an optimal view of the area of the operation. For this purpose, the resectoscope 10 has an eyepiece 24 at a proximal end, which is connected to the optics 15. Alternatively, it is also conceivable that a camera is arranged on the resectoscope 10 instead of the eyepiece 24.

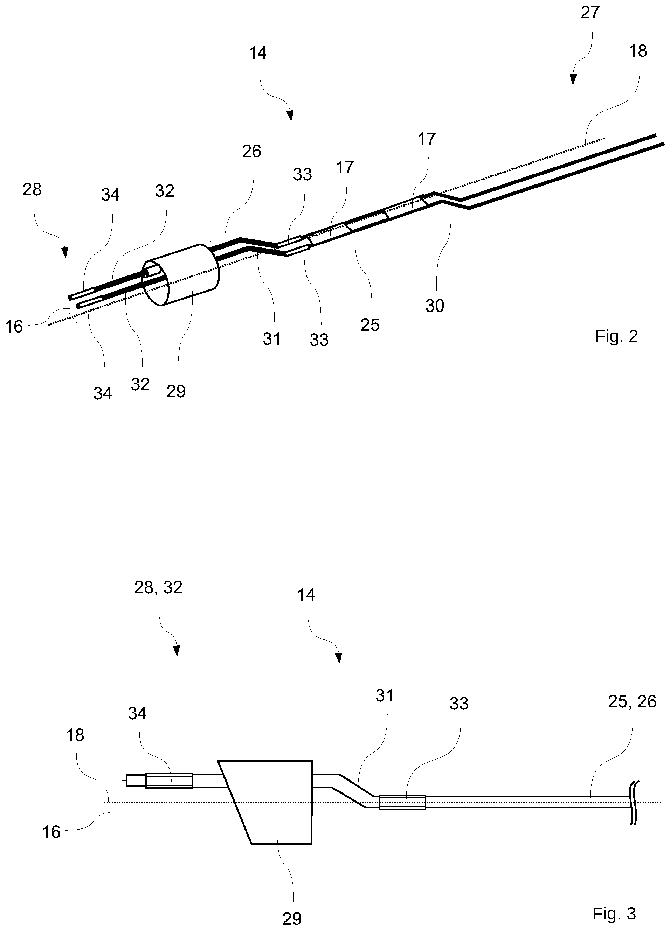

The electrode support 14 consists essentially of two parallel electrode support tubes 25, 26 (Fig. 2). These electrode support tubes 25, 26 serve to hold the electrode 16 and to supply it with electrical energy. For this purpose, an electrical conductor runs in at least one of the electrode support tubes 25, 26 from the proximal end 27 to the distal end 28. One of the two electrode support tubes 25, 26 is latched with the proximal end 27 in the carriage 20 and connected to a cable, which is in contact with an RF generator. The other electrode support tube 25, 26 is also locked into the carriage 20 and forms the neutral electrode. For further stabilization of the elongated electrode support tubes 25, 26, these can be connected to each other via guide elements 17. These guide elements 17 also serve to clamp the electrode support 14 on the inner shaft13 or under the inner shaft 13 or the optics 15. In addition, the electrode support tubes 25, 26 are guided by an insulating insert 29 and are thereby also stabilized against transverse forces.

For the resectoscope to function properly, it is essential that the electrode support 14 together with the electrode 16 can be completely drawn into the outer shaft 12. For this purpose, the effective cross-section or the outer cross-section of the electrode 16 must not be larger than the inner diameter of the distal region of the outer shaft 12. Accordingly, it is known that the electrode support tubes 25, 26 have an S-curvature 30. This S-curvature 30 causes two parallel, successive sections along the electrode support tubes 25, 26 to be displaced in parallel relative to the longitudinal axis 18 so that the distal ends 28 of the electrode support tubes 25, 26 are offset from the longitudinal axis 18. In order to optimize the shape of the electrode 16 and to increase the space inside the shaft 13, the invention provides that the electrode support tubes 25, 26 comprise a second S- curvature 31. As a result of this second S- curvature 31, two distal portions 32 of the electrode support tubes 25, 26 are spaced even further from the longitudinal axis 18 than as already provided by the S-curvature 30. Furthermore, the invention provides that the distal sections 32 of the electrode support tubes 25, 26 have an oval cross-section in contrast to the other sections of the electrode support tube 25, 26. This oval cross-section is formed such that a height of the cross-section perpendicular to a horizontal plane is greater than a width of the cross-section. This section with the oval cross-section includes both the distal section 32 and the S-curvature 31. The remaining sections of the electrode support tubes 25, 26 further have a circular cross-section. For a defined transition of the sections with a circular cross-section to the sections with an oval cross-section, the electrode support tubes 25, 26 each have a crimp 33 or an embossing or a deformation. This crimp 33 also fixes the electrical conductor within the electrode support tubes 25, 26 (Fig. 3).

In addition, the invention also contemplates that the distal ends of the electrode support tubes 25, 26 have a crimp 34 or an embossment or a deformation. This hexagonally formed crimp 34 is oriented such that two parallel side surfaces of the crimp 34 extend transversely to a horizontal plane through the electrode support tubes 14. As a result, the cross-section of the crimp 34 behaves similarly to the oval cross-section of the electrode support tubes 25, 26. Thus, the electrode support tubes 25, 26 can be completely retracted without the crimp 34 becoming jammed in the insulating insert 29. At this point, it should be expressly pointed out that the insulating insert 29 in Figs. 2 and 3 is shown in a highly schematized form and is merely for illustrative purposes.

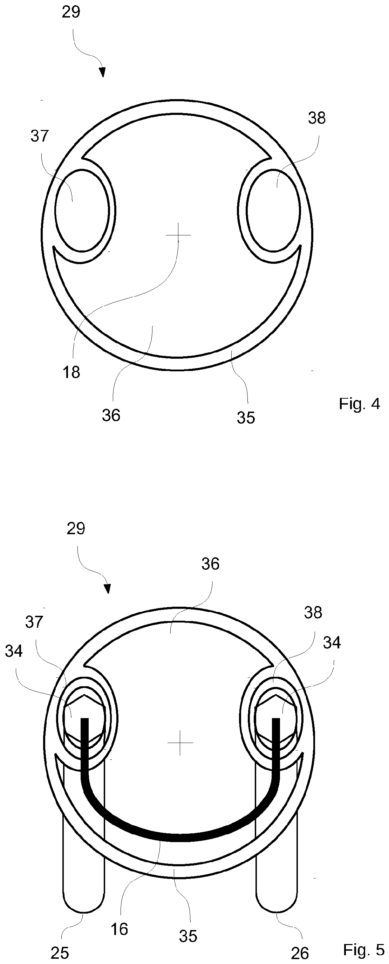

Figs. 4, 5 show a front view of the insulating insert 29 according to the invention. In order to prevent the electrode 16, to which an electrical voltage is applied, from coming into contact with the metallically conductive outer shaft 12, it is known to arrange an electrically insulating tip at the distal tip of the inner shaft 13. This tip, which is usually tubular, is made of an electrically non-conductive material, such as a plasma- and temperature-stable plastic. The insulating insert 29 can be releasably coupled to the shaft 13 by means of coupling elements.

The insulating insert 29 according to the invention, shown here schematically, is also tubular and has a thin wall 35. This wall 35 comprises a central passage 36 through which, for example, the optics 15 and other tools can be guided. Furthermore, the insulating insert 29 has two bores 37, 38. These bores 37, 38 are aligned parallel to each other as well as parallel to the longitudinal axis 18, but are displaced upwards relative to the longitudinal axis 18, so that the bores 37, 38 do not lie centrally or in the same horizontal plane as the longitudinal axis 18. The bores 37, 38 serve to receive the two electrode support tubes 25, 26. For receiving the electrode support tubes 25, 26, the bores 37, 38 are also of oval design, the height of the bores 37, 38 being greater than their width. Only due to the oval design of the bores 37, 38, the oval cross-section of the electrode support tubes 25, 26 and due to the second S-curvature 31 is it possible to displace the two bores 37, 38 relative to the longitudinal axis 18 and, in fact, without having to change the distance between the two bores 37, 38. As a result, the electrode 16 can also retain its known width and does not have to be changed. Rather, this reshaping or displacement makes it possible to increase the effective cross-section of the electrode 16 by maintaining its width and adapting the length or height of the electrode 16 to the dimension of the resectoscope 10. Due to the fact that the distance between the two bores 37, 38 has not been changed, previous electrodes 16 can also be used with the electrode support 14.

In Fig. 5, the insulating insert 29 is shown with the two electrode support tubes 25, 26 passing through the two bores 37 and 38. In particular, it is clear that the crimp 34 of the distal ends of the electrode support tubes 25, 26 do not protrude beyond the diameter of the oval cross-sections of the distal sections 32 and that the electrode support tubes 25, 26 are thus freely displaceable through the bores 37, 38 of the insulating insert 29.

### List of Reference Numerals:

- 10: Resectoscope
- 11: Resectoscope shaft
- 12: Outer shaft
- 13: Inner shaft
- 14: Electrode support
- 15: Optics
- 16: Electrode
- 17: Guide element
- 18: Longitudinal axis
- 19: Handle
- 20: Sled
- 21: Handle part
- 22: Handle part
- 23: Spring bridge
- 24: Ocular
- 25: Electrode support tube
- 26: Electrode support tube
- 27: Proximal end
- 28: Distal end
- 29: Insulation insert
- 30: S-curvature
- 31: S-curvature
- 32: Distal section
- 33: Crimping
- 34: Crimping
- 35: Wall
- 36: Passage
- 37: Bore
- 38: Bore

## Claims

1. Insulating insert (29) for a hand-held electrosurgical instrument, in particular for a resectoscope (10), wherein the tube-like insulating insert (29) can be detachably coupled with a proximal end region to a distal end of a tube-like shaft (13) of the hand-held instrument and has a central passage (36) for receiving a shaft, **characterized in that** two bores (37, 38) for receiving a respective electrode support tube (25, 26) of an electrode support (14) are arranged in a wall (35) of the insulating insert (29) opposite and parallel to the central passage (36).

2. Insulating insert (29) for a hand-held electrosurgical device according to claim 1, **characterized in that** the two bores (25, 26) have an oval cross-section, wherein a height of the oval cross-section is greater than a width of the oval cross-section.

3. Insulating insert (29) for a hand-held electrosurgical device according to claim 1 or 2, **characterized in that** the two bores (25, 26) are preferably displaced upwards or downwards with respect to a central longitudinal axis (18) through the insulating insert (29).

4. Insulating insert (29) for a hand-held electrosurgical device according to any of the preceding claims, **characterized in that** the wall (35) of the insulating insert (29) is reinforced around the two bores (37, 38) and is otherwise reduced in wall thickness.

5. Electrode support (14) for an electrode (16) of an electrosurgical hand instrument, in particular for a resectoscope (10), with at least one, preferably two, electrode support tubes (25, 26), wherein at a distal end (28) of the at least one electrode support tube (25, 26), the electrode (16) being arrangeable and the electrode support (14) being couplable to a main body of the hand instrument via a proximal end (27) of the at least one electrode support tube (25, 26), **characterized in that** the at least one electrode support tube (25, 26) has an oval or convex cross-section at least in sections , in particular over an entire length .

6. Electrode support (14) according to claim 5, **characterized in that** a distal portion (32) of the at least one electrode support tube (25, 26) has an oval or convex cross-section.

7. Electrode support (14) according to claim 5 or 6, **characterized in that** the oval cross-section of the distal portion (32) of the at least one electrode support tube (25, 26) has a greater height than width, the height-to-width ratio being 1.1:1 to 1.7:1, preferably 1.4:1, wherein in particular the oval cross-section of the distal section (32) of the at least one electrode support tube (25, 26) has a height of 1.4 mm and a width of 1 mm.

8. Electrode support (14) according to one of claims 5 to 7, **characterized in that** the distal section (32) of the at least one electrode support tube (25, 26) has a length of from 20 mm to 50 mm, preferably from 24 mm to 40 mm, in particular is longer than 30 mm.

9. Electrode support (14) according to any one of claims 5 to 8, **characterized in that** a transition between a proximal portion of the at least one electrode support tube (25, 26) and the distal portion (32) is formed by a embossing or a crimping (33), wherein in particular the crimp (33) has a hexagonal cross-section, wherein two opposing side surfaces of the hexagonal cross-section are oriented perpendicular to a horizontal plane.

10. Electrode support (14) according to any one of claims 5 to 9, **characterized in that** a distal end of the at least one electrode support tube (25, 26) has a preferably hexagonal crimp (34).

11. Electrode support (14) according to one of claims 5 to 10, **characterized in that** the at least one electrode support tube (25, 26) has at least one, preferably two, namely a proximal S-curvature (30) and a distal S-curvature (31), S-curvature (30, 31), wherein by this at least one S-curvature (30, 31) a section of the electrode support tube (25, 26) is displaced in parallel relative to another section of the electrode support tube (25, 26), and wherein preferably two parallel electrode support tubes (25, 26) each have at least one S-curvature (30, 31), the tube sections each being displaced in only one plane and the distal S-curvature (30) lying in the same plane as the proximal S-curvature (31).

12. Electrode support (14) according to claim 11, **characterized in that** the at least one distal S-curvature (31) has a height of 0.2 mm to 2.0 mm, preferably of 0.7 mm, and wherein the at least one distal S-curvature (31) has a length of from 2.0 mm to 20.0 mm, preferably from 5.0 mm to 10.0 mm.

13. Electrode support (14) according to claim 11 or 12, **characterized in that** the distal portion (32) of the at least one electrode support tube (25, 26) having the oval or convex cross-section has the at least one S-curvature (30, 31).

14. Method for manufacturing an electrode support (14) for an electrode (14) of an electrosurgical hand-held instrument with at least one, preferably two, electrode support tubes (25, 26) according to claim 5, **characterized in that** a cross-section of a distal section (32) of the electrode support tube (25, 26) is formed oval (step A) and/or that a transition from a section with a circular to a section with an oval cross-section is stamped or crimped (step B) and/or that the distal area with the oval cross-section is provided with at least one S-curvature (31) (step C).

15. Method for manufacturing an electrode support (14) according to claim 14, **characterized in that** the steps A, B and C are carried out successively or simultaneously.

16. Hand-held electrosurgical instrument, in particular a resectoscope (10), having a main body and at least one tubular shaft which is connected to the main body, it being possible to couple an insulating insert (29) according to Claims 1 to 4 to a distal end of the shaft, and an electrode support (14) according to Claims 5 to 13 extending through the shaft and through the insulating insert (29), which electrode support (14) is fastened to the main body by a proximal end and to the distal end of which an electrode (16) can be arranged.
